# EUROPEAN PATENT SPECIFICATION

(11) **EP 3 922 268 B1**
(45) Date of publication and mention of the grant of the patent: **22.02.2023**
(21) Application number: 21177893.1
(22) Date of filing: 07.06.2021
(51) Int. Cl.: A61K 47/61, A61K 47/55, A61K 47/64, A61P 29/02, A61P 35/00

(54) **NEW DERIVATIVES OBTAINED FROM HYALURONIC ACID AND CARNOSINE**
AUS HYALURONSÄURE UND CARNOSIN GEWONNENE NEUE DERIVATE
NOUVEAUX DÉRIVÉS OBTENUS À PARTIR D'ACIDE HYALURONIQUE ET DE CARNOSINE

(30) Priority: 11.06.2020 IT 202000014017
(43) Date of publication of application: 15.12.2021
(73) Proprietor: Fidia Farmaceutici S.p.A., 35031 Abano Terme (PD) (IT); Universita' Degli Studi Di Catania, 95131 Catania (IT)
(72) Inventor: RIZZARELLI, Enrico, 95124 Catania (CT) (IT); SCIUTO, Sebastiano, 95124 Catania (CT) (IT); GRECO, Valentina, 95124 Catania (CT) (IT); SATRIANO, Cristina, 95124 Catania (CT) (IT); INTURRI, Rosanna, 35031 Abano Terme (PD) (IT); MESSINA, Luciano, 35031 Abano Terme (PD) (IT); VACCARO, Susanna, 35031 Abano Terme (PD) (IT)
(74) Representative: Bianchetti & Minoja SRL

(56) References cited:
- EP-A1- 1 176 154
- WO-A1-2016/016847
- WO-A1-2018/113802
- WO-A1-2019/069258
- IMPELLIZZERI DANIELA ET AL: "Protective effect of a new hyaluronic acid -carnosine conjugate on the modulation of the inflammatory response in mice subjected to collagen-induced arthritis", BIOMEDICINE AND PHARMACOTHERAPY, ELSEVIER, FR, vol. 125, 25 February 2020 (2020-02-25), XP086087309, ISSN: 0753-3322, DOI: 10.1016/J.BIOPHA.2020.110023 [retrieved on 2020-02-25]
- SIRACUSA ROSALBA ET AL: "The Protective Effect of New Carnosine-Hyaluronic Acid Conjugate on the Inflammation and Cartilage Degradation in the Experimental Model of Osteoarthritis", APPLIED SCIENCES, vol. 10, no. 4, 15 February 2020 (2020-02-15), page 1324, XP055776198, DOI: 10.3390/app10041324

## Description

### OBJECT OF THE INVENTION

The object of the invention is the chemical conjugate between carnosine (β-alanyl-L-histidine) and hyaluronic acid covalently bonded via a peptidase-resistant amine bond; said conjugate is therefore characterised by improved resistance to enzymatic degradation. Further objects of the invention are compositions for pharmaceutical, cosmetic and nutraceutical use comprising said conjugate, and the uses thereof.

### FIELD OF INVENTION

The cell respiration process generates an important and potentially dangerous byproduct: the superoxide radical, which can react with and damage biological macromolecules such as proteins, DNA and lipids and generate other highly reactive species (ROS) such as ^{•}OH, RO₂^{•}, ROOH, RO', H₂O₂ and ONOO', which are potentially aggressive towards the cell system. All aerobic organisms possess defence mechanisms against the O₂^{•-} radical, the main ones being superoxide dismutase (SOD) enzymes, which catalyse the dismutation of O₂^{•-} to O₂ and H₂O₂. The hydrogen peroxide produced is in turn converted to H₂O by catalase and by glutathione peroxidase, thus preventing the formation of the ^{•}OH radical which would have originated from the Fenton reaction (Fe(II)+ H₂O₂→ Fe(III) + ^{•}OH + ⁻OH). Under certain pathological conditions, such as inflammatory processes, SOD enzymes can be insufficient to eliminate the excess O₂^{•-}produced, and in such cases, the biological tissues can be further damaged. A high level of small molecules with antioxidant activity, such as glutathione and other peptides (carnosine, homocarnosine, anserine, etc.), which have the function of reinforcing protection against oxidative stress processes, has been found in some vertebrate tissues, such as muscle or brain tissue, characterised by a higher level of oxidative metabolism. The protective activity of said peptides against ROS has been extensively discussed and demonstrated in the literature (for example in Prokopieva et al., Oxid Med Cell Longer*.*

In the case of carnosine in particular, as regards its anti-radical and anti-peroxidative properties, pre-clinical studies have demonstrated its ability to prevent, and partly treat, inflammatory damage, as demonstrated in the tissues of the digestive apparatus, eyes and skin. Moreover, carnosine has exhibited inhibitory effects against oxidation of human LDLs induced by copper Cu(II) (Bellia et al., Eur J Med Chem, 2007, 43:373-380), and its scavenging activity against the hydroxyl radical has been specifically demonstrated (La Mendola et al., Helv Chim Acta, 2002, 85:1633-1643). It is also interesting to note that complexes of carnosine with Cu(II) exhibit synergic activity against two toxic radical species, O₂^{•-} and ^{•}OH (Bonomo et al., Dalton Trans, 2003, 23: 4406-4415). The scavenging activity of said carnosine complexes with Cu(II) is also present when they dimerise (Tamba and Torreggiani, Int J Radiat Biol, 1999, 75:1177-1188). As regards the ability of carnosine to counteract the pathological effects of nitrosative stress, it has been demonstrated that direct interaction with nitric oxide is responsible for the protective effect (Nicoletti et al., J Neurosci Res, 2007, 85:2239-2245). The efficacy of carnosine in reducing PARP-1 and PARP-2 activation after induction of oxidative stress was also demonstrated more recently (Spina-Purrello et al., Neurochem Res, 2010, 35:2144-2153; Bellia et al., Mol Aspects Med, 2011, 32:258-266).

The peptide nature of carnosine involves some limitations on its use, associated with its degradability by specific peptidases called carnosinases, and by other enzymes normally present in biological tissues. The use of N-acetyl-carnosine, which is produced by processes known to the skilled person and purified chromatographically with large amounts of solvents, has been proposed to overcome this limitation (WO9510294). Derivatisation of carnosine with cyclodextrins, compounds which are widely used as drug carriers, by means of a chemical procedure involving successive purification steps using chromatographic techniques, has also been used for the same purpose (EP1176154). A conjugate of carnosine with trehalose was subsequently patented on the basis of the same rationale. The synthesis of the compound requires subsequent purification by cation-exchange column chromatography (EP1860116). However, the exact degradation profile of the derivatised carnosine as disclosed in WO9510294, EP1176154 and EP1860116 by enzymes naturally present in biological tissues is not known, and the real *in vivo* action capacity of said derivatives remains unproven.

Unlike the previous conjugates, the conjugate of formula (1) according to the present invention is obtained by covalently conjugating carnosine with hyaluronic acid (HA). The synthesis procedure of conjugate (1) is simple, as is the subsequent purification, which does not involve chromatographic techniques, merely a dialysis step. Moreover, conjugate (1) is not a target of non-specific peptidases, and exhibits unexpected resistance to degradation by hyaluronidase. Conjugate (1) disclosed herein therefore appears to be simpler to obtain and more stable than carnosine alone, either as acetyl-derivative, or derivatised with trehalose or cyclodextrins. Moreover, the presence of HA in the conjugate according to the present invention enhances the action of carnosine against the oxidative processes described above, improving its biological activity with respect to known compounds.

Hyaluronic acid (HA) is a straight-chain polymer consisting of a variable number of repeating disaccharide units, each composed of residues of glucuronic acid (β-D-glucopyranuronic acid) and acetylglucosamine (2-acetilamino-2-deoxy-β-D-glucopyranose), bonded to one another via a glycoside bond between the anomeric carbon atom of the former and the C-3 of acetylglucosamine. HA is one of the main constituents of the extracellular matrix (ECM) of vertebrate tissues, the presence of which is particularly significant in synovial fluid, vitreous humour and hyaline cartilage, but also abundant in skin, tendons and muscle. HA plays a key role in providing mechanical support for the tissues, as well as supporting a wide variety of cellular processes. It is known that HA, through its CD44 membrane receptor, modulates many different processes relating to cell physiology and biology such as cell proliferation, migration, differentiation and angiogenesis, and also performs other functions such as hydrating the tissues and lubricating the joints. It has also been demonstrated that HA plays a crucial role in the tissue repair process, both from the structural standpoint (by organising the extracellular matrix and regulating its hydration), and as a substance that stimulates a wide range of processes wherein it acts directly and/or indirectly (clot formation, phagocyte activity, fibroblast proliferation, neovascularisation, re-epithelialisation, etc.) (Weigel P. et al., J Theoretical Biol, 1986, 119:219-234; Abatangelo G. et al., J Surg Res, 1983, 35:410-416; Goa K. et al., Drugs, 1994, 47:536-566).

In view of the properties described above, HA is used to treat a wide range of disorders, ranging from wound healing to counteracting joint cartilage degeneration processes. Healing of wounds and ulcers of various kinds, including chronic ones, considerably improves after local application of ointments or unguents containing HA. Indeed, it has been demonstrated that HA protects wound granulation tissue because it prevents the damage deriving from local formation of free oxygen radicals (Trabucchi et al., Int J Tissue React, 2002, 24:65-71). HA can also be incorporated in molecular "scaffolds" which act as templates for tissue regeneration in the field of tissue engineering (Fakhari and Berkland, Acta Biomater, 2013, 9:7081-92). HA is also used as a substitute for vitreous humour in the ophthalmological field, and as dermal filler and moisturising agent in the cosmetic field. One of the most important biomedical uses of HA is undoubtedly intra-articular injection to counteract the cartilage degeneration associated with various disorders, including osteoarthritis (OA) and rheumatoid arthritis (RA). As well as acting as a synovial fluid substitute, HA also acts as an anti-inflammatory, modulating the release of inflammatory cytokines (in particular IL-1), and binds to the dynorphin receptors, giving rise to an analgesic effect (Zavan et al., PLoS One, 2013, 8:e55510).

EP2648715 discloses a compound comprising HA which is salified, or at least partly salified, with carnosine, to obtain preparations offering the biopharmacological properties of both compounds. The compound described is characterised in that the chemical bond between HA and carnosine is the ionic type, and therefore weak and solely electrostatic. Chemical species, or electrolytes, held together by an ionic bond, are well known to dissolve in aqueous solution, immediately releasing carnosine and free HA, which are both rapidly degraded by the action of numerous enzymes normally present therein, including carnosinases, non-specific peptidases and hyaluronidases.

A conjugate between hyaluronic acid (HA) and carnosine has also been prepared wherein the amino group of the peptide is involved in an amide bond with the carboxyl of some of the glucuronic acid units present in the polysaccharide chain (EP3174555). An interesting improvement in resistance to the action of human serum carnosinase (CN-1) has been demonstrated for this conjugate. It has also been disclosed that HA conjugated with carnosine via an amide bond and having a high degree of derivatisation is useful in the treatment of osteoarthritis (OA), rheumatoid arthritis (RA), and a series of disorders directly deriving therefrom (WO2019/069258). However, the amide bond characterising the HA-carnosine conjugates described in EP3174555 and WO2019/069258 is susceptible to the action of hydrolysis by non-specific peptidases, thus considerably limiting the duration of the beneficial effect of said compounds.

The conjugate of formula (1) according to the invention is characterised by the presence, between carnosine and HA, of a strong covalent chemical bond which does not dissolve in aqueous solution as does the ionic bond described in EP2648715. In conjugate (1) described herein, the bond between carnosine and HA is effected by means of an amine bridge, which is not susceptible to hydrolysis by enzymes possessing peptidase activity. The conjugation of HA and carnosine provides unexpectedly greater resistance to degradation by hyaluronidases, thus giving conjugate (1) characteristics superior to those of the compounds described in EP2648715 and EP3174555.

### DETAILED DESCRIPTION OF THE INVENTION

The object of the invention is a derivative of carnosine (β-alanyl-L-histidine) of formula (1), obtained by functionalising hyaluronic acid with carnosine.

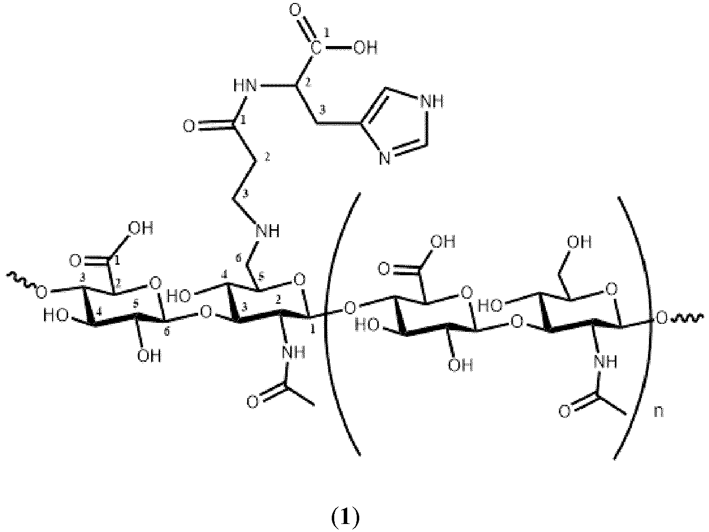

The conjugate of formula (1) of carnosine dipeptide with hyaluronic acid has an amine bond between the free amino group of carnosine and the carbon atom at the 6-position of the acetylglucosamine residue of hyaluronic acid. In formula (1), index n is the number of disaccharide units (each consisting of a glucuronic acid and an acetylglucosamine residue) not conjugated with carnosine which are present in the polysaccharide.

The present invention discloses a conjugate between HA and carnosine having formula **(1)** which can be obtained by known processes. The purification of conjugate **(1)** conveniently does not require chromatography steps; in particular it does not require column chromatography steps, which are expensive and necessitate the use of large amounts of solvents, nor does it require ion-exchange chromatography steps. By way of example, the conjugate **(1)** can be obtained by a preparation process comprising the following steps:
a) regioselective oxidation to aldehyde of one or more primary alcohol functions, present in the amount of 1 per disaccharide unit, bonded to the carbon at the 6-position of each acetylglucosamine residue of HA;
b) formation of an aldimine bond between the free amino group NH₂ of carnosine and one or more aldehyde groups of the aldehyde derivative of hyaluronic acid;
c) conversion *in situ* of the resulting aldimine bridge to an amine bridge using a suitable reducing agent;
d) purification of the product by means of a dialysis step.

The HA used in the present invention can derive from any source, such as rooster combs (EP138572), fermentation (from *Streptococcus equi* or *zooepidemicus,* EP0716688), or biotechnology processes (from *Bacillus,* EP2614088, EP2614087), and can be purified by various techniques (EP3491027, WO2019016699). The HA is preferably obtained by fermentation from *Streptococcus equi* or *zooepidemicus* (EP0716688). The weight-average molecular weight of HA for the applications described herein can range between 1×10⁴ and 3×10⁶ Da, preferably between 1×10⁵ Da and 1×10⁶ Da, and most preferably in the range 130-230 kDa or in the range 500 -750 kDa and mixtures thereof. HA with a weight-average molecular weight (MW) ranging between 130 and 230 kDa is generally abbreviated to "HA with weight average MW of 200 kDa". "Average molecular weight" here means the weight-average MW calculated by the "intrinsic viscosity" method (Terbojevich et al., Carbohydr Res, 1986, 363-377).

Conjugate **(1)** according to the invention is characterised by a degree of derivatisation (i.e. functionalisation) of HA with carnosine by means of formation of the amine bond between the free amino group of carnosine and the carbon atom at the 6-position of the acetylglucosamine residue of HA binding to a primary hydroxyl, which can range from 0.1 to 80%, preferably from 5 to 60%, more preferably from 10 to 30%, and most preferably from 10 to 15%. "Degree of derivatisation" (i.e. functionalisation) here means the percentage of carbon atoms at the 6-position of the N-acetylglucosamine units of HA which were conjugated with carnosine by formation of an amine bridge. For example, a conjugate according to the invention with a 3% degree of derivatisation will have 3 acetylglucosamine residues conjugated with carnosine every 100 disaccharide units present in the polysaccharide.

A further object of the invention is the conjugate of formula **(1)** in the form of a copper Cu(II) complex. Copper has angiogenic and antibacterial properties, and SOD-like activity in complexes with carnosine. Both conjugate **(1)** and its complex with Cu(II) exhibit low susceptibility to degradation by the enzyme hyaluronidase, and a greater ability to stimulate cell viability than that of carnosine alone.

Conjugate **(1)** according to the invention is characterised by improved resistance to enzymatic degradation, in particular by peptidase and hyaluronidase. Carnosine, remaining in its conjugated form, is resistant to the action of serum carnosinase. Moreover, increased resistance by conjugate **(1)** to degradation by hyaluronidase has been demonstrated, with the result that conjugate **(1)** keeps carnosine bound and intact for a sufficient time for it to reach its site of action. Conjugate **(1)** is therefore more stable than the hyaluronic acid derivatives with carnosine previously described, allowing a synergic action between HA and carnosine, as will be demonstrated below.

The invention also relates to pharmaceutical, cosmetic or nutraceutical compositions comprising the conjugate of formula **(1)** as active ingredient. In view of their antioxidant, wound-healing and chelating properties towards transition metals, compositions comprising the conjugate of formula **(1)** can be used for the treatment or prevention of various pathological conditions, including disorders characterised by oxidative damage to cells and tissues: the conjugate of formula **(1)** can be used in the treatment and/or prevention of disorders characterised by oxidative damage such as cancer, cardiac and neurological disorders, ophthalmic disorders, complications of diabetes such as diabetic retinopathy, and lesions of the internal organs such as gastric ulcers. The use of conjugate **(1)** has also proved advantageous in the treatment of pathological conditions characterised by the presence of chronic wounds, ulcers, cuts, burns or abrasions, wherein wound healing needs to be promoted. Conjugate **(1)** is also useful in the prevention and/or treatment of inflammatory states of the joints resulting from degeneration thereof, in particular in the prevention and treatment of osteoarthritis (OA) and treatment of rheumatoid arthritis (RA), as well as in the treatment of disorders caused directly by RA or indirectly correlated with/dependent on RA.

Pharmaceutical, cosmetic or nutraceutical compositions comprising the conjugate of formula **(1)** as active ingredient can take various pharmaceutical forms, including solutions, ointments, powder, gel, ophthalmic drops and the like. Said pharmaceutical, cosmetic or nutraceutical compositions can be advantageously formulated with excipients, stabilisers, viscosity-controlling agents and/or preservatives as known to the skilled person, for topical and ophthalmic, oral, intra-articular or parenteral application, or surgically applicable at the site of treatment. In particular, compositions comprising conjugate **(1)** can be used in the field of advanced dressings for healing wounds and ulcers, including chronic ones. For this purpose, compositions comprising conjugate **(1)** can be used to impregnate or can be deposited on gauze, bandages, adhesive dressings, patches, or three-dimensional structures or scaffolds of various kinds. They can be deposited by conventional or innovative methods, such as methods that exploit the known plasma technology.

The doses and methods of administration depend on a number of factors which will generally be determined by the skilled person on the basis of the pharmacological properties of conjugate **(1).** The doses will be advantageously lower than those normally required for carnosine, in view of the greater activity and lower susceptibility to enzymatic degradation exhibited by conjugate **(1)** and its Cu(II) complexes.

Unless otherwise indicated, commercially available reagents were used in all the examples described.

### EXAMPLE 1. Synthesis of hyaluronic acid (200 kDa)-carnosine conjugate with a 10% degree of derivatisation.

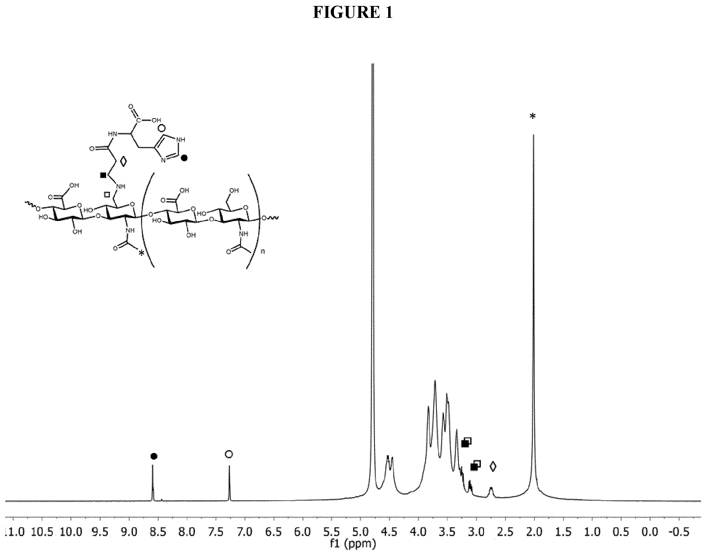

***Trial design.*** In a typical synthesis process, 2,2,6,6-tetramethylpiperidine-1-oxyl (TEMPO; 4.12 mg) and trichloroisocyanuric acid (TCCA; 122.6 mg) were added in sequence to a solution of hyaluronic acid (200 kDa) (1 g) dissolved in 100 ml DMF/water (70:30, v/v) and, after adjusting the pH to 8 with 0.1N NaOH, the resulting mixture was left at room temperature for 60 min. 3 ml of methanol was added to quench the reaction, and the subsequent addition of 1 L of acetone caused the precipitation of the polysaccharide component. 1 g of the resulting aldehyde derivative of hyaluronic acid was dissolved in 100 ml of water, previously adjusted to pH 8 by adding a saturated aqueous solution of sodium bicarbonate. Then, 179 mg of carnosine (0.79 mmols) and, after 30 minutes, 497.4 mg (7.9 mmols) of the reducing agent sodium cyanoborohydride were added to said solution. The resulting mixture was then left at room temperature for 12 hours. After said time, the reaction mixture was dialysed against water for 48 h using a dialysis membrane with MWCO of 12-14 kDa. The dialysate was recovered and freeze-dried to provide the expected conjugate (985 mg), which was subjected to spectroscopic analysis. The ¹H-NMR spectrum was recorded on a Varian instrument at 500 MHz (Inova 500), using as reference frequency that of HOD, and is shown in **FIGURE 1****.**

***Analysis of results**.* In the ¹H-NMR spectrum of the amino-conjugate (D₂O) illustrated in **FIGURE 1** are present the signals relating to the protons of the imidazole ring at 8.62 and 7.30 ppm, while the anomeric protons and those of the methine group of the histidine residue overlap between 4.60 and 4.45 ppm. Other signals belonging to protons of the polysaccharide chain fall into the region between 3.3 and 4.0 ppm. In addition to the signals at 3.8 and 3.9 ppm, corresponding to the diastereotopic protons of the methylene group at the 6-position of the non-derivatised acetylglucosamine residue, the spectrum shows signals at 2.78 and 3.15 assignable to the methylene analogues (at the 6-position of the acetylglucosamine residue) which are now involved in the formation of the amine bridge of the conjugate. This shift to higher fields confirms the formation of the covalent bond between the amino group of carnosine and the carbon atom at the 6-position of the acetylglucosamine residue of hyaluronic acid. The signals of the diastereotopic protons in α at the amino group of the β-alanine residues overlap with those of the methylene groups at the 6-position of the glucosamine residues, while the protons of the methylene groups in α at the carbonyl group of the β-alanine residues produce a signal at 2.75 ppm. The amount of carnosine conjugated with hyaluronic acid was calculated on the basis of the ratio between the peak area relating to the protons of the imidazole ring and the peak area corresponding to the protons of the methyl groups of the acetylglucosamine units. The degree of derivatisation resulted to be 10%.

### EXAMPLE 2. Preparation of copper complexes of hyaluronic acid and HA-carnosine conjugate.

***Trial design.*** In a typical procedure for preparation of copper complexes, the lyophilisates of HA and of the HA-carnosine conjugate, prepared as described in EXAMPLE 1, were dissolved at the concentration of 10 mg/mL in ultrapure Millipore water (resistivity 18.2 MΩ·cm at 25°C, TOC values below 5 ppb). To ensure complete solubilisation of the polymer, the samples are maintained under stirring for 2 h at room temperature (23°C). The pH is then adjusted to 7.4, and copper sulphate (CuSO₄) is added to obtain the following final concentrations in copper ions: 1.36·10⁻³ M, 2.72·10⁻³ M and 5.44·10⁻³ M, corresponding to [Cu(II)]: [carnosine] molar ratios in the conjugate of 1:2, 1:1 and 2:1 respectively. Finally, the pH is checked again, and corrected if necessary to the value of 7.4.

### EXAMPLE 3. Enzymatic hydrolysis by ovine testicular hyaluronidase.

***Trial design.*** The stability of the Cu(II) copper complexes of conjugate (1) towards ovine testicular hyaluronidase (HyAse) was evaluated by enzymatic hydrolysis assay. Phosphate buffer (23 mM, with the addition of 150 mM NaCl) at pH = 6.4 was used to solubilise the enzyme (HyAse concentration = 70 mg/ml) and to dissolve hyaluronic acid weighing 200 kDa, used as control, and conjugate (1), prepared as described in EXAMPLE 1 (hyaluronic acid concentration, either alone or conjugated with carnosine, amounting to 2 mg/ml; carnosine concentration in the conjugate amounting to 5.44·10⁻⁴ M). To prepare the Cu(II) complexes, copper sulphate CuSO₄, at the concentration [Cu(II)] = [carnosine] = 5.44·10⁻⁴ M, was added to solutions of hyaluronic acid and conjugate (1) in phosphate buffer, using a procedure similar to the one described in EXAMPLE 2. 25 µl of HyASE was added to 250 µl of each sample to give 4000 IU of enzyme in the reaction medium. After incubation at 37°C for 4 hours under stirring (300 rpm in Eppendorf ThermoMixer), the enzymatic reaction was interrupted by adding potassium tetraborate (50 µl of a 2.8 M solution prepared in KOH 1.39 M) followed by heat shock (5 min at 100°C, then 2 min at 0°C). Spectrophotometric analysis of the oligosaccharides produced following enzymatic digestion with HyAse of the Cu(II) complexes of conjugate (1) or free hyaluronic acid was conducted by colorimetric determination, according to the procedure described by Schanté and colleagues (Schanté et al., Carbohydrate Polymers, 2011, 85:469-89), which uses the Morgan-Elson reaction. The purple product obtained from the reaction was analysed by UV-vis spectroscopy, measuring the absorption peak at the wavelength of 586 nm. All the reagents used were obtained from Sigma Aldrich. Immediately before the start of the Morgan-Elson reaction, 750 µl of a solution of dimethylaminobenzaldehyde (DMAB, prepared by dissolving 200 mg of the product in 250 µl of HCl and 4.75 ml of CH₃COOH) was added to the resulting solution. After 60 min incubation at the temperature of 37°C in a Thermomixer (300 rpm), the UV-vis spectra were recorded with a Beckman DU 650 or Lambda2S spectrophotometer (Perkin Elmer, USA). The Morgan-Elson reaction produces a purple product which exhibits an absorption peak at 586 nm, indicative of degradation of hyaluronic acid by the HyAse enzyme. All the measurements were conducted at 25 ± 0.2°C using cuvettes with an 0.5 cm optical path.

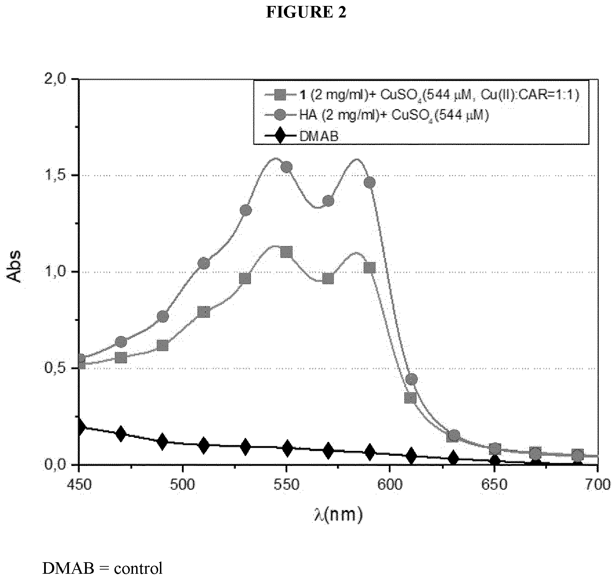

***Analysis of results**.* The recorded spectra are shown in **FIGURE 2****,** where it can be noticed that the spectrum corresponding to the degraded product deriving from the Cu(II) complex of conjugate **(1)** shows a lower intensity than the spectrum corresponding to the Cu(II) complex of HA at all the wavelengths λ considered, in particular in the interval including the absorption peak of the purple product found, which ranges from 550 to 600 nm. As the Beer-Lambert law states that the absorbance detected at a certain wavelength A_{λ}, and concentration c of the chemical species whose spectrum is recorded, are directly proportional to one another, it can be concluded that the level of degradation found for λ between 550 and 600 nm of the Cu(II) complex of conjugate **(1)** is about 35% lower than that of the Cu(II) complex of HA, after exposure to the hyaluronidase enzyme. This finding therefore demonstrates that conjugate **(1)** degrades about 35% in less than non-conjugated HA in the presence of hyaluronidase.

### EXAMPLE 4. Effects of conjugate (1) on cell viability.

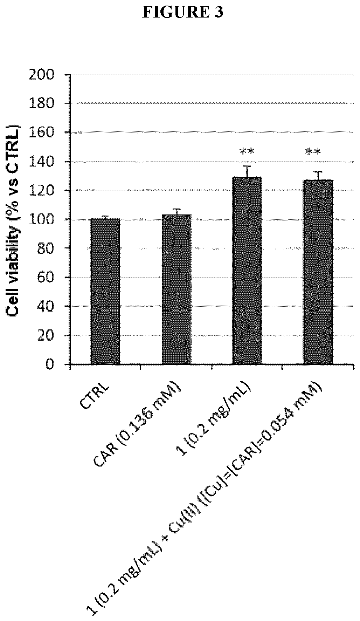

***Trial design.*** The cell response of conjugate (1) in terms of cell viability was determined *in vitro* by performing the MTT assay (Mosmann T, J Immunol Methods 1983; 65:55-63) on the HFF1 cell line (consisting of fibroblasts derived from human skin). The cells were used at passages 20-23, cultured at 37°C and 5% CO₂ in Dulbecco's Modified Eagle Medium (DMEM) with integrated glucose and added with FBS (1%). To conduct the MTT assay, the cells were plated in 96-well multiwell plates (7000 cells per well) in DMEM culture medium with integrated glucose and added with 1% FBS; after 48 hours they were treated with control medium, or with medium alternatively containing carnosine, conjugate **(1)** prepared as described in EXAMPLE 1, or complexes of conjugate **(1)** with Cu(II), for 5 hours of incubation at 37°C and 5% CO₂. Each treatment was performed in triplicate. To prepare the Cu(II) complexes tested in this experiment, copper sulphate CuSO₄ was used at the concentration of [Cu(II)] = [carnosine] = 5.44 · 10⁻⁵ M, corresponding to a hyaluronic acid concentration in conjugate (1) of 0.2 mg/ml. When the incubation time had elapsed, each of the wells containing the cells was treated with 100 µL of 3-2,5-diphenyltetrazolium bromide reagent (5 mg/ml in DMEM) for 90 min. After removal of the liquid from each well and addition of DMSO (100 µl), the absorbance of the formazan salt (blue) produced by the cleavage reaction of the tetrazolium ring of MTT (yellow) by the mitochondrial enzyme succinate dehydrogenase present in the viable cells only was measured in a multiplate reader (Synergy^{™} 2 Multi-Mode Microplate Reader) at the wavelength of 570 nm. The results are set out in **FIGURE 3****.**

***Analysis of results**.* As shown in the graph in **FIGURE 3****,** MTT analysis demonstrates that conjugate **(1)** gives rise to an increase in cell viability. Said increase in viability compared with the control is significant and of considerable size, amounting to just over 25% in the case of conjugate **(1)** and the complexes thereof with Cu(II), thus demonstrating the synergy between hyaluronic acid and carnosine. Conversely, treatment of the same cells with carnosine alone did not lead to any increase in cell viability.

### Brief description of figures

FIGURE 1: ¹H NMR spectrum of HA-carnosine conjugate **(1),** in D₂O, wherein both the signals characterising the conjugate (■, □, ◊) and the signals used to calculate the percentage loading of carnosine (∘, ●, ∗) are shown.
FIGURE 2: Stability to hyaluronidase of the Cu(II) complexes of conjugate **(1)** (in red, symbol: square) and non-conjugated HA (in blue, symbol: circle) evaluated by the Morgan-Elson reaction of dimethylaminobenzaldehyde (DMAB) with the oligosaccharide products of enzymatic degradation, which produces a purple product that exhibits an absorption peak at a 586 nm wavelength. The absorption of the DMAB reagent alone is shown in the graph (in black, symbol: diamond) as experiment control.
FIGURE 3: Cell viability measured by MTT assay (spectrophotometric measurement of absorbance, at the wavelength of 570 nm, of the formazan solution formed by reacting 3-2,5-diphenyltetrazolium bromide with the mitochondrial enzyme succinate dehydrogenase, which plays a key role in cell metabolism) on HFF1 cells incubated for 5 hours with control medium (CTRL), or medium alternatively containing carnosine (0.136 mM, i.e. 1.36·10⁻⁴ M), conjugate **(1)** (at the concentration of 0.2 mg/mL, or the Cu(II) complexes thereof at the ratio [carnosine]:[Cu] = 1:1). The values shown are the mean ± S.E.M. of each experiment, conducted in triplicate. Statistical significance calculated with the one-way ANOVA test (** = p < 0.01, vs CTRL).

## Claims

1. Conjugate of formula **(1),** 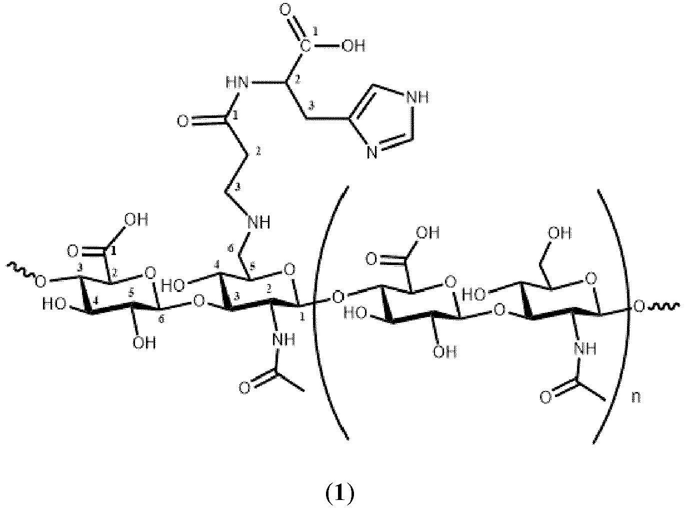 of carnosine dipeptide with hyaluronic acid by formation of an amine bond between the free amino group of carnosine and the carbon atom at the 6-position of the acetylglucosamine residue of hyaluronic acid.

2. A conjugate according to claim 1, wherein the hyaluronic acid has a weight average molecular weight ranging between 1×10⁴ and 3×10⁶ Da, preferably between 1×10⁵ Da and 1×10⁶ Da, and most preferably in the range 130-230 kDa or in the range 500 -750 kDa and mixtures thereof.

3. Conjugate according to claims 1-2, wherein the degree of derivatisation of hyaluronic acid, by formation of the amine bond with carnosine, ranges from 0.1 to 80%, preferably from 5 to 60%, more preferably from 10 to 30%, and most preferably from 10 to 15%.

4. Conjugate according to one or more of the preceding claims, wherein the conjugate of formula **(1)** is in the form of a Cu(II) complex.

5. Pharmaceutical, cosmetic or nutraceutical compositions, comprising the conjugate of formula **(1)** according to claims 1-4 as active ingredient.

6. Compositions according to claim 5, in the form of solutions, ointments, powder, gel or ophthalmic drops.

7. Compositions according to claim 5, for topical, ophthalmic, oral, intra-articular, parenteral or surgical application.

8. Compositions according to claim 5, used to impregnate or deposited on gauze, bandages, adhesive dressings, plasters, or three-dimensional structures or scaffolds.

9. Compositions according to any one of claims 5-8, for use in the treatment and/or prevention of disorders **characterised by** oxidative damage, such as cancer, cardiac and neurological disorders, ophthalmic disorders, complications of diabetes such as diabetic retinopathy, and lesions of internal organs such as gastric ulcers.

10. Compositions according to any one of claims 5-8, for use in the treatment of chronic wounds, ulcers, cuts, burns or abrasions.

11. Compositions according to any one of claims 5-8, for use in the prevention and treatment of osteoarthritis (OA) and in the treatment of rheumatoid arthritis (RA).

## Patentansprüche

1. Konjugat der Formel (1) 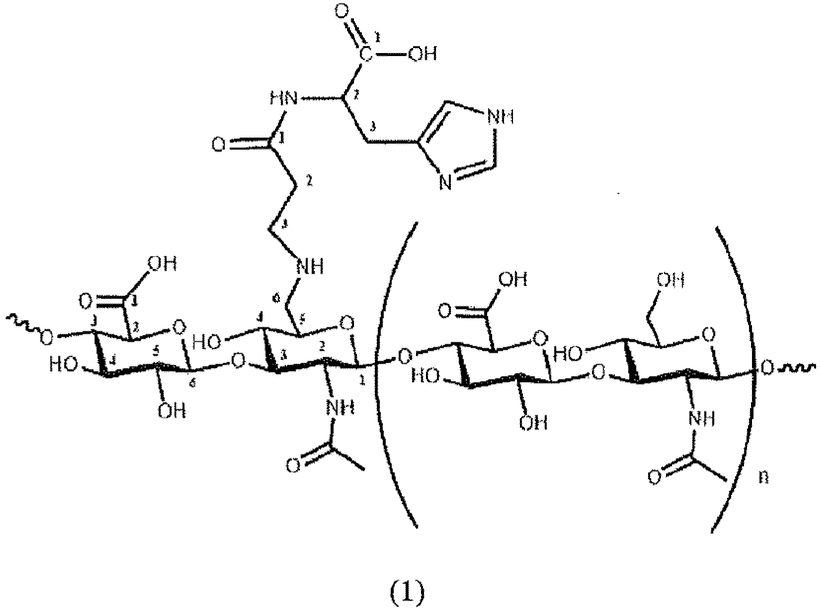 von Carnosindipeptid mit Hyaluronsäure durch Bildung einer Aminbindung zwischen der freien Aminogruppe von Carnosin und dem Kohlenstoffatom an Position 6 des Acetylglucosaminrests von Hyaluronsäure.

2. Konjugat gemäß Anspruch 1, wobei die Hyaluronsäure ein gewichtsmittleres Molekulargewicht hat, das zwischen 1×10⁴ und 3×10⁶ Da liegt, vorzugsweise zwischen 1×10⁵ Da und 1×10⁶ Da, und am stärksten bevorzugt in dem Bereich 130-230 kDa oder in dem Bereich 500-750 kDa, sowie Gemische davon.

3. Konjugat gemäß Anspruch 1-2, wobei der Derivatisierungsgrad von Hyaluronsäure durch Bildung der Aminbindung mit Carnosin von 0,1 bis 80%, vorzugsweise von 5 bis 60%, stärker bevorzugt von 10 bis 30%, und am stärksten bevorzugt von 10 bis 15% reicht.

4. Konjugat gemäß einem der vorangehenden Ansprüche, wobei das Konjugat der Formel (1) in der Form eines Cu(II)-Komplexes vorliegt.

5. Pharmazeutische, kosmetische oder nutrazeutische Zusammensetzungen, umfassend das Konjugat der Formel (1) gemäß Ansprüchen 1-4 als Wirkstoff.

6. Zusammensetzungen gemäß Anspruch 5 in der Form von Lösungen, Salben, Pudern, Gelen oder Augentropfen.

7. Zusammensetzungen gemäß Anspruch 5 zur topischen, ophthalmischen, oralen, intraartikulären, parenteralen oder chirurgischen Anwendung.

8. Zusammensetzungen gemäß Anspruch 5, verwendet zum Imprägnieren oder abgeschieden auf Gaze, Verbänden, selbsthaftenden Wundverbänden, Pflastern oder dreidimensionalen Strukturen oder Scaffolds.

9. Zusammensetzungen gemäß einem der Ansprüche 5-8 zur Verwendung in der Behandlung und/oder Vorbeugung von Erkrankungen, die durch oxidative Schädigung gekennzeichnet sind, wie zum Beispiel Krebserkrankung, kardiale und neurologische Erkrankungen, Augenerkrankungen, Diabeteskomplikationen, wie zum Beispiel diabetische Retinopathie, sowie Verletzungen innerer Organe, wie zum Beispiel Magengeschwüre.

10. Zusammensetzungen gemäß einem der Ansprüche 5 bis 8 zur Verwendung in der Behandlung von chronischen Wunden, Geschwüren, Schnittverletzungen, Verbrennungen oder Abschürfungen.

11. Zusammensetzungen gemäß einem der Ansprüche 5 bis 8 zur Verwendung in der Vorbeugung und Behandlung von Osteoarthrose (OA) sowie in der Behandlung von rheumatoider Arthritis (RA).

## Revendications

1. Conjugué de formule (1), 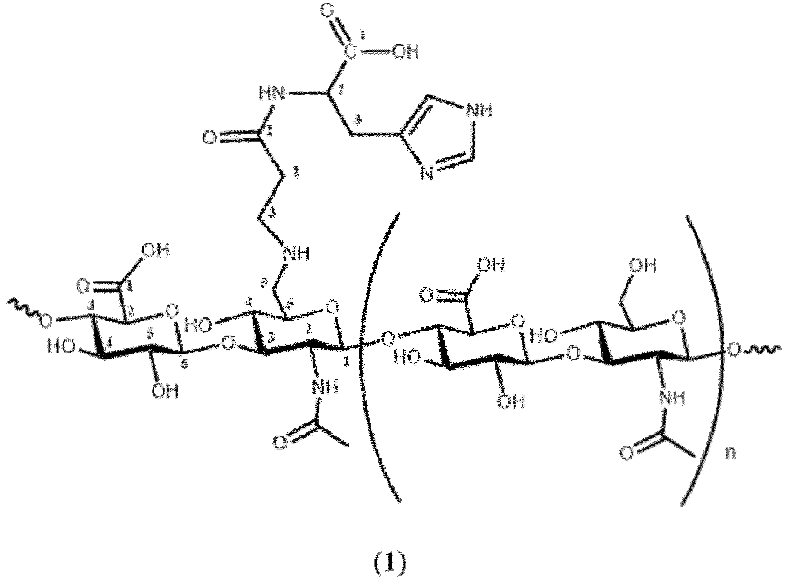 du dipeptide carnosine avec de l'acide hyaluronique par formation d'une liaison amine entre le groupe amino libre de la carnosine et l'atome de carbone en position 6 du résidu acétylglucosamine de l'acide hyaluronique.

2. Conjugué selon la revendication 1, dans lequel l'acide hyaluronique a un poids moléculaire moyen en poids compris entre 1×10⁴ et 3×10⁶ Da, de préférence entre 1×10⁵ Da et 1×10⁶ Da, et de manière préférée entre toutes dans la plage de 130 à 230 kDa ou dans la plage de 500 à 750 kDa et leurs mélanges.

3. Conjugué selon les revendications 1 à 2, dans lequel le degré de dérivatisation de l'acide hyaluronique, par formation de la liaison amine avec la carnosine, va de 0,1 à 80 %, de préférence de 5 à 60 %, plus préférablement de 10 à 30 %, et de manière préférée entre toutes de 10 à 15 %.

4. Conjugué selon une ou plusieurs des revendications précédentes, dans lequel le conjugué de formule (1) est sous la forme d'un complexe de Cu(II).

5. Compositions pharmaceutiques, cosmétiques ou nutraceutiques, comprenant le conjugué de formule (1) selon les revendications 1 à 4 en tant que principe actif.

6. Compositions selon la revendication 5, sous forme de solutions, de pommades, de poudre, de gel ou de gouttes ophtalmiques.

7. Compositions selon la revendication 5, pour application topique, ophtalmique, orale, intra-articulaire, parentérale ou chirurgicale.

8. Compositions selon la revendication 5, utilisées pour imprégner ou être déposées sur des gazes, des bandages, des pansements adhésifs, des plâtres, ou des structures ou échafaudages tridimensionnels.

9. Compositions selon l'une quelconque des revendications 5 à 8, pour une utilisation dans le traitement et/ou la prévention de troubles **caractérisés par** des dommages oxydatifs, tels que le cancer, des troubles cardiaques et neurologiques, des troubles ophtalmiques, des complications du diabète telles que la rétinopathie diabétique, et des lésions des organes internes telles que les ulcères gastriques.

10. Compositions selon l'une quelconque des revendications 5 à 8, pour une utilisation dans le traitement de plaies chroniques, d'ulcères, de coupures, de brûlures ou d'écorchures.

11. Compositions selon l'une quelconque des revendications 5 à 8, pour une utilisation dans la prévention et le traitement de l'arthrose (OA) et dans le traitement de la polyarthrite rhumatoïde (RA).
